(19) Europäisches Patentamt
European Patent Office
Office européen des brevets

(11) EP 0 633 786 B1

(12) EUROPEAN PATENT SPECIFICATION

(45) Date of publication and mention of the grant of the patent:
**01.08.2001 Bulletin 2001/31**

(21) Application number: **93903538.2**

(22) Date of filing: **27.01.1993**

(51) Int Cl.[7]: **A61K 41/00**, A61L 2/08, A61M 1/36

(86) International application number:
**PCT/US93/00401**

(87) International publication number:
**WO 93/14791 (05.08.1993 Gazette 1993/19)**

# (54) METHOD OF INACTIVATION OF VIRAL, PARASITIC AND BACTERIAL BLOOD CONTAMINANTS

VERFAHREN ZUR INAKTIVIERUNG VON VIRALEN, PARASITÄREN UND BAKTERIELLEN BLUTVERUNREINIGUNGEN

PROCEDE D'INACTIVATION DES CONTAMINANTS VIRAUX, PARASITAIRES ET BACTERIENS DU SANG

(84) Designated Contracting States:
**AT BE CH DE DK ES FR GB GR IE IT LI LU MC NL PT SE**

(30) Priority: **27.01.1992 US 825691**

(43) Date of publication of application:
**18.01.1995 Bulletin 1995/03**

(73) Proprietor: **BAXTER INTERNATIONAL INC.**
**Deerfield, Illinois 60015 (US)**

(72) Inventors:
- **GOODRICH, Raymond, P., Jr.**
  **Pasadena, CA 91106 (US)**
- **YERRAM, Nagendar**
  **South Pasadena, CA 91030 (US)**
- **HACKETT, Roger, W.**
  **Pasadena, CA 91107 (US)**
- **WAALKES, Marjan van Borssum**
  **NL-3906 ZK Veenendaal (NL)**

(74) Representative: **Lucas, Brian Ronald**
**Lucas & Co.**
**135 Westhall Road**
**Warlingham Surrey CR6 9HJ (GB)**

(56) References cited:

| | |
|---|---|
| **EP-A- 0 124 363** | **EP-A- 0 196 515** |
| **EP-A- 0 457 196** | **WO-A-91/16060** |
| **WO-A-93/00005** | **US-A- 4 402 318** |

Remarks:
The file contains technical information submitted after the application was filed and not included in this specification

EP 0 633 786 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

**Description**

FIELD OF THE INVENTION

This invention relates to inactivating viral/bacterial/parasitic contamination of liquid, lyophilized, reconstituted blood cell compositions comprising erythrocytes, platelets, etc, or protein fractions.

BACKGROUND OF THE INVENTION

A major concern in the use of stored or donated homologous blood or plasma protein preparations derived from human blood is the possibility of viral and bacterial contamination.

Viral inactivation by stringent sterilization is not acceptable since this could also destroy the functional components of the blood, particularly the erythrocytes (red blood cells) and the labile plasma proteins. Viable RBC's can be characterized by one or more of the following: capability of synthesizing ATP; cell morphology; $P_{50}$ values; oxyhemoglobin, methemoglobin and hemichrome values; MCV, MCH, and MCHC values; cell enzyme activity; and in vivo survival. Thus, if lyophilized then reconstituted and virally inactivated cells are damaged to the extent that the cells are not capable of metabolizing or synthesizing ATP, or the cell circulation is compromised, then their utility in transfusion medicine is compromised.

There is an immediate need to develop protocols for the deactivation of viruses that can be present in the human red blood supply. For example, only recently has a test been developed for Non A, Non B hepatitis, but such screening methods, while reducing the incidence of viral transmission, do not make the blood supply completely safe or virus free. Current statistics indicate that the transfusion risk per unit of transfused blood is as high as 1:100 for Non A, Non B hepatitis, and ranges from 1:40,000 to 1:1,000,000 for HIV, depending on geographic location. Clearly, it is desirable to develop a method which inactivates or removes virus indiscriminately from the blood.

Contamination problems also exist for blood plasma protein fractions, such as plasma fractions containing immune globulins and clotting factors. For example, new cases of non A, non B hepatitis have occurred in hemophilia patients receiving protein fractions containing Factor VIII which have been treated for viral inactivation according to approved methods. Therefore, there is a need for improved viral inactivation treatment of blood protein fractions.

SUMMARY OF THE INVENTION

The present invention thus provides a process for reducing viral, bacterial and/or parasitic contaminants in a composition comprising blood, a blood component, cell culture or a component of a cell culture, comprising the steps of:

mixing said composition in a liquid state with a chemical radiation sensitizer capable of targeting said viral, bacterial and/or parasitic contaminants;
treating the composition with an organic solvent or detergent; and
exposing the composition in the liquid or lyophilized state to electromagnetic radiation of a wavelength and intensity and for a period of time sufficient to activate said sensitizer, whereby the activation of the sensitizer reduces said contamination in said composition.

The present invention is applicable to viral or bacterial decontamination of dried (lyophilized or evaporatively dried), frozen, liquid or reconstituted cells (erythrocytes, platelets, haemosomes and other cellular or cell-like components), recombinant protein preparations, blood protein fractions, other blood components including red cells, platelets and leukocytes, stem cells, protein solutions or other frozen compositions intended for subsequent *in-vivo* use such as plasma derived factors. The present invention involves utilization of sensitizers which bind selectively to a viral nucleic acid, coat protein or membrane envelope. The sensitizer is also a moiety which can be activated upon exposure to radiation, which may be in the form of ultra-violet radiation, but preferably is in the form of ionizing radiation such as X-rays or gamma-rays which can penetrate the sample containing the contamination. While not intending to be limited to a particular theory, in frozen cell or protein containing compositions, some of the water is present in the form of ice but there is also unfrozen water trapped in a highly viscous glassy state. Water molecules which are present in these glassy states have low mobility and may possibly form hydroxy radicals which can randomly damage cells. However, due to the low mobility in the glassy state, damage to cells from these hydroxy radicals is reduced. Therefore, by irradiating a frozen suspension of cells containing the sensitizers, random damage of the cells due to the hydroxy radicals may be avoided due to the inability of the sensitizer to migrate in the frozen suspension and the inability of the hydroxy radicals to form and migrate through the frozen suspension. In this manner, damage is localized on the targeted viral or bacterial particle. sensitizing compounds for the treatment of tumors is provided in "Cancer: Principles and Practice of Oncology" second edition, J.B. Lippincott Company, V.T. DeVita, Jr., S. Hellman and S.A. Rosenberg,

editors, 1985, pp. 2256-2279.

The invention allows for the cells or protein fractions to be useful in a transfusable state, while still maintaining relatively high cell viability, ATP synthesis and oxygen transport, in the case of cellular components, and therapeutic efficacy, in the case of protein fractions.

The lyophilization and reconstitution media described below may be utilized to lyophilize and reconstitute proteins, particularly, blood plasma protein fractions. The protein fraction may be virally/bacterially deactivated by mixing with a chemical sensitizer, lyophilized (freeze-dried) or frozen, then irradiated. If the lyophilization media of the invention is used, it is contemplated that the constituents of the media also serve to provide some degree of protection of the dry proteins during irradiation.

A preferred embodiment comprises reducing viral and bacterial contamination of dried or reconstituted cells rehydrated with washing solutions containing a polymer or mixture of polymers having a molecular weight in the range 1K to 360 K. All molecular weights, throughout this specification are in Daltons. Thus "K" = KiloDaltons.

This is followed by one or more additional wash cycles using a wash of a dextrose-saline solution at a pH in the range of about 7.0-7.4. The dextrose-saline solution will also contain a polymer having a molecular weight in the range of about 1K to 40K, and preferably about 2.5K. The composition of reconstituted cells will also preferably contain a monosaccharide.

Preferably the cells will have been previously lyophilized using a lyophilization solution buffered in the range of pH of 7.0 to 7.4 preferably by a phosphate-buffered solution. A typical phosphate-buffered lyophilization solution will comprise mono- and di-basic potassium and sodium phosphate (usually in the range of 1-10 mM each) and 5-10 mM adenine. This solution maintains the pH at around 7.2.

A preferred phosphate-buffered solution to be used as the lyophilization buffer will comprise nicotinic acid, reduced glutathione, glutamine, inosine, adenine, monopotassium phosphate, magnesium chloride disodium phosphate all of which will serve as a basic salt buffer at a pH of about 7.2. In addition this lyophilization buffer will contain a final concentration of about 26% weight by volume of a monosaccharide, preferably 1.7 M glucose, and a final concentration of about 3.0% weight by volume of polyvinylpyrrolidone (average molecular weight of 360K), and a final concentration of about 15% weight by volume of hydroxyethyl starch (average molecular weight of 500K).

The term lyophilization is broadly defined as freezing a substance and then reducing the concentration of the solvent, namely water, by sublimation and desorption, to levels which will no longer support biological or chemical reactions. Usually, the drying step is accomplished in a high vacuum. However, with respect to the storage of cells and particularly erythrocytes, the extent of drying (the amount of residual moisture) is of critical importance in the ability of cells to withstand long-term storage at room temperature. Using the procedure described herein, cells may be lyophilized to a residual water content of less than 10 weight %, preferably less than 3%, and still be reconstituted to transfusable, therapeutically useful cells. Cells with about 3 weight % water content using this procedure may be stored for up to two weeks at room temperature, and at 4°C for longer than eight months, without decomposition. This far exceeds the current A.A.B.B. standard for refrigerated storage of red blood cells of six weeks at 4°C or less than one day at room temperature without decomposition. These dried cells may be deactivated using a chemical sensitizer described herein.

According to a preferred embodiment of the present invention, washed packed red blood cells are mixed with a chemical sensitizer, then washed to remove excess sensitizer not bound to viral or bacterial nucleic acid, and the treated cells are then lyophilized. The dry cell and sensitizer mixture will then be irradiated, typically with gamma radiation, at an intensity of about 3K-50K rads, for a period of time sufficient to destroy viruses (in particular, the single-stranded or double-stranded RNA/DNA viruses), without any substantial adverse effect on the recovery and usefulness of the cells. Other wavelengths of electromagnetic radiation such as visible light or X-rays, may be used.

In another preferred embodiment, the chemical sensitizers may be added to liquid protein preparations, then lyophilized and irradiated. Particularly preferred are blood protein preparations, including but not limited to, plasma proteins, blood protein extracts, clotting factors (such as Factors VIII and IX), immune globulins and serum albumin.

Dry (lyophilized) cells or protein fractions may be directly mixed with the chemical sensitizer, then irradiated.

From the foregoing description, it will be realized that the invention can be used to selectively bind a metal atom or a metal atom containing chemical sensitizer to blood-transmitted viruses, bacteria, or parasites. Also monoclonal or polyclonal antibodies directed against specific viral antigens (either coat proteins or envelope proteins) may be covalently coupled with either a metal atom or a metal atom-containing sensitizer compound, thereby increasing the effective cross-section of the contaminant to penetrating or other forms of radiation energy.

Since cell compositions also comprise a variety of proteins, the method of decontamination of cells described herein is also applicable to protein fractions, particularly blood plasma protein fractions, including, but not limited to, fractions containing clotting factors (such as Factor VIII and Factor IX), serum albumin and/or immune globulins. The viral and bacterial inactivation may be accomplished by treating a protein fraction with a sensitizer as described herein. A protein fraction which has been lyophilized and reconstituted may be sensitized and irradiated to deactivate possible contamination. It is contemplated that liquid and frozen protein fractions may also be decontaminated according to the

present invention.

The process of the invention may also be applied to cell cultures comprising a recombinent plasma protein, for example recombinant serum albumin or recombinant clotting factor, especially factor VIII.

Depending upon the nature of the presumed radiolytic mechanism of the sensitizer reaction with the virus, other types of radiation may be used, such as X-ray, provided the intensity and power utilized is sufficient to inactivate the viral contamination without adverse effect on the cells. Mature human red blood cells and platelets lack nucleic acids, therefore the nucleic acid binding sensitizers selectively target contaminating viruses and bacteria. Although described in connection with viruses, it will be understood that the methods of the present invention are generally also useful to inactivate any biological contaminant found in stored blood or blood products, including bacteria and blood-transmitted parasites. Furthermore, the present may be used to inactivate viruses or viral particles for the preparation of vaccines.

## BRIEF DESCRIPTION OF THE DRAWING

The Figure is a plot of viral reduction in plasma according to the procedure of Example 1, to show the effect of the sensitizer.

## DETAILED DESCRIPTION OF THE INVENTION

The cells are preferably prepared by immersing a plurality of erythrocytes, platelets and/or hemosomes, etc. in a physiologic buffered aqueous solution containing a carbohydrate, and one or more biologically compatible polymers, preferably having amphipathic properties. By the term amphipathic it is meant there are hydrophobic and hydrophilic portions on a single molecule. This immersion is followed by freezing the solution, and drying the frozen solution to yield novel freeze-dried erythrocytes containing less than 10%, and preferably about 3% or less by weight of moisture, which, when reconstituted, produce a significant percentage of viable, transfusably useful red blood cells, platelets or hemosomes. Preferred methods of reconstitution of the lyophilized composition are described below. Although described in connection with red blood cells, it will be understood that the methods are generally also useful to lyophilize platelets, hemosomes, and blood protein fractions.

The carbohydrate utilized to prepare erythrocyte, platelet and/ or hemosome compositions according to the invention is biologically compatible with the erythrocytes, platelets or hemosomes, that is, non-disruptive to the cells or hemosome membrane, and one which permeates, or is capable of permeating, the membrane of the erythrocytes, platelets or hemosomes. It is also advantageous to stabilize proteins, especially labile blood proteins, with the carbohydrates during lyophilization and irradiation according to the invention. The carbohydrate may be selected from the group consisting of monosaccharides, since disaccharides do not appear to permeate the membrane to any significant extent. Monosaccharide pentoses and hexoses are preferred as is a final concentration of from about 7.0 to 37.5 weight % in phosphate buffered saline (PBS) or a phosphate buffered solution, preferably about 26%. Xylose, glucose, ribose, mannose and fructose are employed to particular advantage.

It will be understood that the cells may be lyophilized using other protocols and irradiated as described below. Although viral inactivation will be attained, the advantage of retaining a significant percentage of viable useful red blood cells is lost if the described lyophilization procedure is not followed.

The invention will be hereafter described in connection with erythrocytes (RBC's) but it will be understood it is also applicable to platelets, hemosomes or other blood cell types or biological cells, as well as protein fractions, particularly plasma protein fractions.

The erythrocytes will preferably be prepared from whole blood centrifugation, removal of the plasma supernatant and resuspending the cells in PBS or another phosphate buffered solution or a commercial dextrose-saline solution. This wash cycle may be repeated 2-3 times preferably using a commercial dextrose-saline solution, then the packed cells are diluted with the lyophilization buffer described above so that the final diluted concentration of carbohydrate and polymer are maintained in the necessary ranges.

Alternatively, commercially available packed blood cells may be used, which typically are prepared in CPDA (commercial solution containing citrate, phosphate, dextrose and adenine).

Upon lyophilization to a moisture content of less than 10%, and preferably less than 3%, the lyophilized cells may be maintained under vacuum in vacuum-tight containers, or under nitrogen or other inert gas, at room temperatures for extended periods of time in absence of or without significant degradation of their desirable properties when reconstituted for use as transfusable cells. In using the preferred lyophilization method disclosed herein, a particular advantage of the present invention is that the lyophilized cells may be stored at room temperature for extended periods of time, thus obviating the need for low temperature refrigeration which is required for storing liquid CPDA preserved red blood cells prepared by methods of the prior art. The present invention also obviates the need for very low temperature (-80°C) frozen storage of red blood cells in glycerol.

By using the preferred reconstitution method disclosed herein it is a further advantage that the lyophilized red blood cells may be reconstituted at normal temperatures, i.e. greater than about 4°C up to about 37°C, which corresponds to normal human body temperature, and preferably at room temperature (about 22°C). The reconstitution medium is preferably a solution comprising a polymer or mixture of polymers having a molecular weight of from about 2.5K to 360 K, preferably 5K to about 360K, present in a concentration in the range of about 12 to 30% weight by volume. This polymer may be the same polymer utilized to lyophilize the red blood cells as described above. Hence the polymers polyvinylpyrrolidone, hydroxyethyl starch, and dextran are particularly preferred and most preferred is polyvinylpyrrolidone (preferably molecular weight about 10K) present in a concentration of about 19% weight by volume in the reconstitution solution. The reconstitution solution will be buffered again typically by phosphate-buffered solution comprising monopotassium phosphate and disodium phosphate as described hereinabove to maintain a pH within the range of about 7.0 to 7.4. The most particularly preferred polymer is polyvinylpyrrolidone of an average molecular weight of about 10K. The most preferred reconstitution buffer will also contain adenosine triphosphate (ATP) in a final concentration of about 5mM.

The polymers may be present in the various solutions from a final concentration of about 3.6K weight % up to saturation, and have a molecular weight in the range of from about 2.5K to about 360K. Preferably, the polymers have molecular weights in the range of from about 2.5K to about 500K, most preferably from about 2.5K to 50K, and are present in a concentration of from about 3.6 weight % up to the limit of solubility of the polymer in the solution. Polymers selected from the group consisting of polyvinylpyrrolidone (PVP) and polyvinylpyrrolidone derivatives, and dextran and dextran derivatives provide significant advantages. Most preferred is the use of polyvinylpyrrolidone (an amphipathic polymer) of average molecular weight in the range of 2.5-360K in an amount in the range of 3-20% weight by volume in the solution prior to lyophilization.

Amino acid based polymers (i.e., proteins), dextrans or hydroxyethyl starch may also be employed. In the lyophilization buffer hydroxyethyl starch (M-HES) with an average molecular weight of about 500K is employed in a 15% weight by volume final concentration. Other amphipathic polymers may be used, such as poloxamers in any of their various forms. The use of the carbohydrate-polymer solution in the lyophilization of red blood cells allows for the recovery of intact cells, a significant percentage of which contain biologically-active hemoglobin.

The most preferred reconstitution buffer will be a solution comprising monopotassium phosphate, disodium phosphate and ATP, all of which form a basic salt buffer at a pH of about 7.2, which also contains about 19% weight by volume of polyvinylpyrrolidone (average molecular weight about 10K).

The reconstitution solution may also optionally contain a monosaccharide, preferably present in the concentration range of about 7.0 to 37.5% weight by volume. The preferred monosaccharides are xylose, glucose, ribose, mannose and fructose.

In the most preferred embodiment, the lyophilized erythrocytes can be reconstituted by mixing with an equal volume of the reconstitution buffer at a temperature of about 37°C and mixed. By "equal" it is meant that the volume is the same as the starting volume prior to lyophilization. After initial reconstitution, the solution is preferably diluted 1:1 with 1-4 additional volumes of the reconstitution buffer at a temperature of about 37°C with added mixing until fully hydrated.

Then, it is preferred that the rehydrated cells be washed according to the following procedure. It is realized, however, that once the cells are reconstituted with reconstitution buffer they are in a hydrated and useful form, but the combination of washings described hereinafter are preferred, specifically for clinical purposes.

After separating the cells from the reconstitution buffer by centrifugation, the resulting packed cells are preferably resuspended at room temperature in (approximately the volume used in the initial reconstitution) a wash buffer comprising nicotinic acid, inosine, adenine, glutamine, and magnesium chloride, all present at about 0.4-10mM further comprising sodium chloride and potassium chloride each at about 30mM, buffered by 10mM disodium phosphate to pH 7.2. This wash buffer further comprises a monosaccharide, preferably glucose at a concentration of about 20mM, and a polymer, preferably polyvinylpyrrolyidone, of a molecular weight 40K and present at a concentration of about 16% weight by volume. Separation by centrifugation completes the first post-rehydration step, a washing step.

After the washing step the rehydrated cells may be suspended in a dextrose-saline transfusion buffer at room temperature which preferably contains polyvinylpyrrolidone at a 10% weight by volume final concentration, with an average 2.5K molecular weight. The cells can be used as they are or be returned to autologous plasma. Additional wash steps in a phosphate-buffered diluent buffer can further remove viruses, but this step is optional for preparation of rehydrated, transfusible cells.

The reconstitution and washings described above will in most instances achieve about 4 log reduction of any viral and bacterial contamination, where 1 log reduction is achieved by drying and 3 log reduction is achieved by washing. Of course, different viruses may respond differently, potentially resulting in more than 4 log reduction of contamination.

The reconstituted cells have characteristics which render them transfusable and useful for therapeutic purposes in that their properties are similar to that of fresh (i.e. not previously lyophilized) red blood cells. Typically reconstituted red blood cells according to the present invention have an oxyhemoglobin content greater than about 90% of that in normal red blood cells. Hemoglobin recovery prior to any washing step is typically in the range of 80 to 85%. The overall cellular hemoglobin recovery including the post-hydration washing steps is about 20 to 30%. The morphology of the reconstituted cells according to the present invention (by scanning electron microscope) typically shows no holes or gaps, and primarily discocytic with some stomatocytic morphology. The oxygen carrying capacity of fresh red blood cells (as measured by $P_{50}$, the oxygen partial pressure at which 50% of the oxygen molecules are bound) was measured to be in the range of about 26 to 28 (average 26.7); with an average Hill coefficient (a measure of the cooperative binding of oxygen molecules to native hemoglobin) of 1.95. The typical $P_{50}$ for erythrocytes lyophilized and reconstituted according to the present invention is about 27.5 (average) with an average Hill coefficient of 2.08. Assays of ATP in the reconstituted cells indicate ATP levels suggesting normal ATP to ADP metabolism. Normal hemagglutination by readily available blood typing antisera of red blood cells made according to the present invention is also typically found.

This lyophilization and reconstitution procedure advantageously and significantly diminishes viral/ bacterial contamination in cell-like material (such as hemosomes), and protein fractions. The contamination can be further reduced by the radiation sensitizing and treatment, particularly while the cells or protein fractions are in the dry state.

The starting packed red blood cells or proteins (which may initially be in a liquid or lyophilized state) are mixed with a sufficient amount (based on total wet weight of cells) of a chemical sensitizer. Preferably, in a composition of packed red blood cells (about 10% hematocrit) about 0.1 to 1 mg of the chemical sensitizer will be used per ml of packed cells. Preferably, the mixture will be irradiated with gamma radiation in the range of 3K-50K rads, typically about 3K rads. Preferred exposure is from 1-10 minutes, if using gamma radiation. Alternatively, UV light (320 nm) may be used, particularly for protein fractions. Preferred exposure is from 1-10 minutes, preferably 3 minutes, if using UV radiation. By this irradiation in presence of a sensitizer, there will be about a 6 log reduction of viral and bacterial contamination, based on contamination present prior to washing and irradiation.

The present invention provides a selective method of generating free radicals derived from chemical sensitizers only in the vicinity of viral RNA or DNA. Indiscriminate radiolysis of blood containing virus in a hydrated state produces hydroxyl radical. However, the hydroxyl radical will damage both the red blood cells and associated proteins as well as the viral target. Thus, viral inactivation would be achieved at the sacrifice of red cell viability. Therefore, sensitizers which bind to DNA, RNA, viral coat proteins, and/or viral membranes and which can be selected to generate radicals upon irradiation, are required. Since the radiolysis can be performed in the dry state (preferably less than 10% residual moisture), generation of hydroxyl radicals from water is greatly reduced. In this manner indiscriminate radical damage is further prevented. Exemplary compounds include:

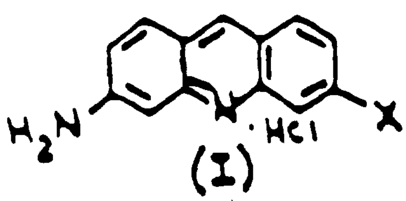

(I)

X = $-N_3$, -I

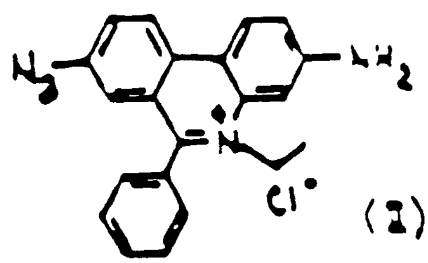

(I)

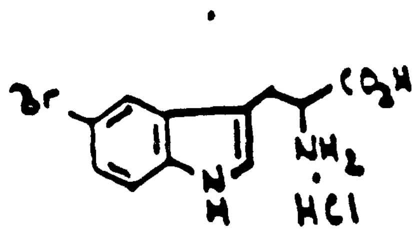

(III)

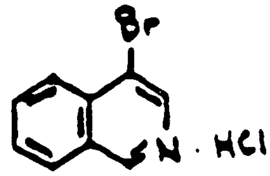

(IV)

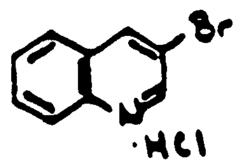

(V)

The preparations of these compounds are known. See Martin, R.F. and Kelly, D.P., Aust. J. Chem., 32, 2637-46 (1979); Firth, W., and Yielding, L.W., J. Org. Chem., 47, 3002 (1982). Other radical-generating reagents which generate radicals upon irradiation are disclosed by Platz et al., Proc. SPIE-Int. Soc. opt. Eng. 847, 57-60 (1988) and Kanakarajan et al., JACS 110 6536-41 (1988).

The radiation-sensitizing compound (which may also be modified to bear a metal atom substituent) may also be selected from the class consisting of DNA-binding drugs, including, but not limited to, netropsin, BD peptide (a consensus peptide from HMG-1), S2 peptide, and the like. These and other DNA-binding drugs are disclosed in Pjura, P.E., Grzeskowiak, K. and Dickerson, R.E. (1987), J. Mol. Biol. 197, 267-271; and Tengi, M., Usman, N., Frederick, C. A. and Wang, A.H.J. (1988), Nucleic Acids Res. 16, 2671-2690.

The radiation sensitizing compound (which may also bear a metal atom) can also comprise a class of DNA-binding proteins and/or polypeptides and/or peptides. Examples of this class of DNA-binding proteins and/or polypeptides and/or peptides are disclosed in Churchill, M.E.A. and Travers, A.A. (1991) Trends in Biochemical Sciences 16, 92-97. Specific examples of DNA-binding peptides include the SE peptide and BD peptide disclosed in the reference herein.

Another class of sensitizers comprises the positively charged porphorins and phthalocyanaines, which bind DNA and RNA. These sensitizers can be activated by irradiation with visible light (500-700 nm).

The DNA-binding specificity can be achieved by covalently coupling the radiation sensitizing compound and/or metal atom to either a DNA-binding drug or to a DNA-binding protein or polypeptide or peptide.

VI

Netropsin

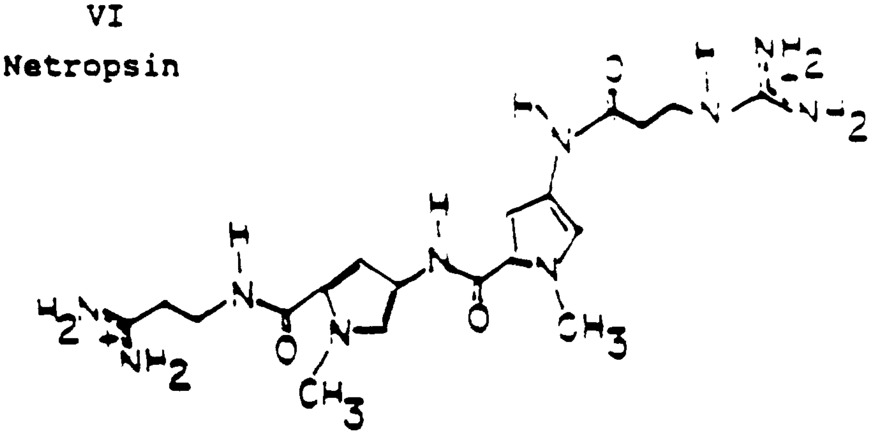

VII

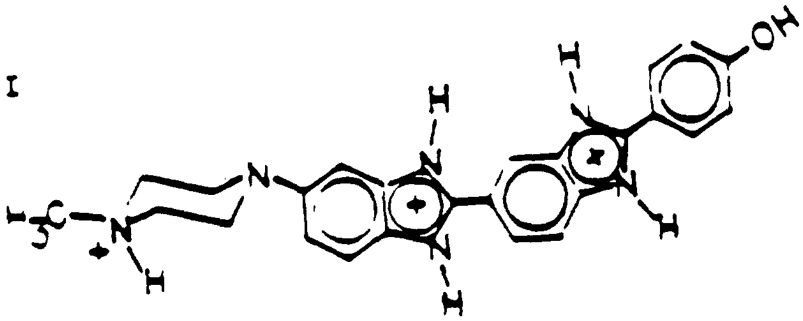

Other sensitizers include specially designed molecules which form triplex DNA, such as those disclosed by Youngquist and Dervan PNAS 82 2565 (1985); Van Dyke and Dervan, Science 225 1122 (1984); Van Dyke and Dervan, Nuc. Acids Res. 11 5555 (1983); Barton and Raphael, PNAS 82 6460 (1985); Barton et al., JACS 106 2172 (1984); and Barton, PNAS 81 1961 (1984). These molecules bind to DNA and RNA, site specifically, if desired, and carry reactive moieties which can generate free radicals in the proximity of the DNA or RNA.

$$R\text{-}I + e^{-} \rightarrow R \bullet I^{-}$$

$$R\text{-}I^{+\bullet} + \text{Guanine} \rightarrow R\text{-}I + \text{Guanine}^{+\bullet}$$

While not intending to be bound by a theory, it is believed that the ejected electron will be captured by that site with the most favorable electron affinity, which is most likely a second molecule of sensitizer elsewhere in the sample. Electron capture by R-I (or R-Br) leads to dissociation of RX with the formation of a radical. The radical so generated will abstract a C-H hydrogen atom from a sugar moiety of a nearby nucleic acid which in turn will lead to DNA or RNA cleavage and viral inactivation.

The radical cation of the sensitizer ($R\text{-}X^{+\bullet}$) will eventually abstract an electron from that component of the sample with the most favorable oxidation potential. This is most likely guanine. The electron transfer reaction forms guanine radical cation. This substance will react with $O_2$ upon reconstitution with aerated $H_2O$. This process also leads to DNA cleavage and viral inactivation. Unreacted material and reaction by-products will be removed during the washing steps involved in the reconstitution of the lyophilized cells (Table 2). This process will also further remove any virus not inactivated by the treatment described above.

Compounds (1) and (2) bind tightly to DNA and RNA by either intercalation and/or by electrostatic interactions between positively charged ammonium ion groups and the negatively charged phosphate groups of the nucleic acid target. Red blood cells do not contain nucleic acids and accordingly will not bind to such compounds by intercalation.

The best mode for using the invention is to add the sensitizer to potentially contaminated blood solutions, and to expose to gamma radiation or x-rays. Fluid solutions of blood are preferably exposed to 3000 rads, and dried lyophilized solid formulations are preferably exposed to 10,000 rads of radiation. It is known that the red cells will survive these doses of radiation in the absence of a sensitizer. Lyophilized blood can withstand higher dosage levels of radiation than hydrated blood.

The gamma radiation or x-rays will be absorbed primarily by the heavy atom of the sensitizer, which will be bound to viral DNA or RNA. The radiation will ionize the sensitizer as follows:

$$R\text{-}I + \gamma\text{-Ray} \rightarrow R\text{-}I^{+\bullet} + e^{-}$$

(X-Ray)

In some instances, particularly if the sensitizer and red blood cells are allowed to stand together for more than several minutes, sensitizers may diffuse into the red blood cells prior to lyophilization. Antioxidants such as glutathione (an excellent hydrogen atom donor) may be added to the preparation to augment the red cell defenses against free radical initiated damage. It will be understood that incorporation of the sensitizer into cells will also allow inactivation of intracellular viruses, especially viruses thought to reside inside white blood cells (most packed red blood cell units contain residual white cells), or intracellular blood parasites, such as malaria parasite which infects red blood cells.

The sensitizers are removed from the reconstituted blood serum or protein fraction by the washing protocol described above for lyophilized cells.

It is preferred that gamma or X-ray radiolysis take place in a dried lyophilized blood (or protein), virus, and sensitizer formulation rather than in a wet, fluid material for several reasons. Firstly, the dry material is less sensitive to radiation and can be exposed to larger doses of $\gamma$-rays or other penetrating radiation without damage to red blood cells (Table 1). This increases the extent of radiolysis of the sensitizer. Secondly, sensitizer radicals bound to DNA or RNA in the dry state can not dissociate from the virus due to the lack of diffusion in the solid material. This will force the sensitizer radical to react with viral RNA or DNA. Thirdly, the solid state conditions will enhance hydrogen atom transfer reactions of the sensitizer radical with the viral nucleic acid, perhaps by quantum mechanical tunneling. Fourthly, the reconstitution and washing protocol used with lyophilized blood or protein fraction serves as a means to remove unreacted material or reaction by-products, and further removes any virus not affected by the treatment.

Other types of radiation may be used including ionizing radiation in general, such as X-ray radiation. Preferably such molecules increase the overall mass attenuation coefficient of the sensitizer to radiation from a pre-determined X-ray target source. In one embodiment a metal and/or halogen atom may be a substituent on a chemical radiation sensitizer molecule which binds to nucleic acids, thereby targeting the bacteria, parasites and viruses. Metal and halogen atom substituents of chemical sensitizers for this purpose include Pt, Br, I, Zn, Cl, Ca and F. The X-ray source may be a metallic target source comprising Mo, Pd, Rh, Ag, W, Ti, Cr, Mn, Fe, Co, Ni, Cu or Zn. It is preferably a tunable source, so that the radiation may be confined to a narrow wavelength and energy band, if so desired. The tunable feature allows for optimization of energy absorption by the metal atoms, thereby directing the absorbed penetrating radiation energy to the production of radicals by a chemical sensitizer bound to nucleic acid.

A preferred metal to be used as the radiation sensitizer is platinum. Another preferred group comprises the halogens, bromine, iodine, chlorine and fluorine. Based on their increasing ability to interact with impinging X-rays, the order of enhancement of radiation sensitizing is expected to be platinum, which is much greater than bromine, which is much greater than iodine, chlorine and fluorine, all of which are much greater than hydrogen.

Compounds containing these atoms, when exposed to X-rays or other forms of ionizing radiation, are capable of forming a reactive species which can interact with the viral nucleic acid, coat protein or lipid envelope, thus destroying it and rendering it non-infectious. This process may be most effective in a dry state where quenching and side reactions due to the presence of water are avoided; however, the procedure will also be applicable in hydrated systems.

In the present invention the sensitizers are utilized in conjunction with solvent detergent systems. Such detergents are known to decrease the viral titre of plasma or separated plasma fractions, presumably by dissolution of the viruses. Such detergents are preferably non-ionic. They include, Tween®, sodium cholate, sodium deoxycholate, Triton®. The organic solvents include tri-n-butyl phosphate and common organic solvents such as ether. Reduction of viral titre by use of these solvent detergents is described for example by Horowitz, *et al*., <u>Transfusion, 25</u>, (6), 516-522 (1985), and <u>25</u>, (6), 523-527 (1985); and U.S. Patent No. 4,946,648. The level of reduction by such solvent detergents may vary as reported in literature to a reduction of one log to greater than five logs of viral titre for such viruses as VSV, Sindbis, and Sandai. The present invention may enhance the reduction of viral titre by these solvent detergents when used in conjunction with the sensitizers and exposure to radiation as set forth herein. While not intending to be bound by a theory, it is believed that the solvent detergents act on the viral proteins or lipid membranes to denature or alter them in a manner which makes them more susceptible to the actions of the sensitizers through the changes induced by the detergents.

A particularly preferred class of sensitizers comprise DNA intercalators, such as hydroxyl, amino methyl, or methyl substituted psoralens, which may be added to plasma or plasma fractions followed by UV radiation to reduce the viral contamination therein. The substituted psoralens are described in U.S. Patent No. 4,727,027 wherein a reduction of about 4 to 7 logs of viral contamination was obtained with extended exposure to ultraviolet radiation. The proposed mechanism of action is to form a photoadduct between the sensitizer and the DNA or RNA of viral origin, which results in loss of infectivity of the virus. According to the present invention, the reduction of viral contamination can be unexpectedly reduced by utilizing brominated psoralens or other halogenated psoralens. For example, it was observed that the bromopsoralens are about 200,000 times more effective in reducing viral activity when compared to use of their non-brominated counterparts. While not intending to be bound by any theory, it is believed that the mechanism of action of the brominated psoralens may be a free radical generation in the proximity of the DNA or RNA resulting in damage of vital nucleic acids of viruses.

The brominated psoralens are in an improvement over the known psoralens and other substituted psoralens when used as sensitizers because the brominated psoralens are an improvement because only one photon of light is required to activate the brominated sensitizer whereas two photons are required to activate a non-brominated sensitizer. Secondly, a brominated psoralen is effective in virtually every intercalative site, whereas a non-brominated sensitizer is effective only in intercalation sites containing a uracil or thymine on different strands of the DNA or RNA. Thirdly, the brominated psoralens may be activated by X-rays as well as UV light.

The use of the brominated or halogenated psoralens is particularly useful in activation in hydrated systems

such as plasma, immune sera, tissue culture media containing animal serum or serum components (such as fetal calf serum), or recombinant products isolated from tissue culture media.

Other types of intercalators may be utilized besides the psoralens and substituted psoralens such as those listed below. These intercalators may be used to target viruses or other blood contaminants. Thus, halogenated or metal atom-substituted derivatives of the following compounds may be utilized as sensitizers:

dihematoporphyrin esters
hematoporphyrin derivatives
benzoporphyrin derivatives
hydrodibenzoporphyrin dimaleimade

hydrodibenzoporhyrin
dicyano disulfone
tetracarbethoxy hydrodibenzoporhyrin
tetracarbethoxy hydrodibenzoporhyrin dipropionamide

The above compounds in their non-halogenated or non-metal atom substituted forms are disclosed in U.S. Patent Nos. 4,649,151, 4,866,168, 4,883,790, 5,053,423 and 5,059,619. When modified with halogen atoms or metal atoms, the above-identified classes of compounds may be sensitized with electromagnetic radiation, including visible light.

The above compounds are included in a class named lipophilic dyes which include dyes such as merocyanine 540 and phthalocyanine derivatives. Merocyanine 540 has been disclosed as useful for the treatment of cancer and viral inactivation of blood cells and plasma proteins (Sieber, *et al*., Photo Chem. and Photo Biology, 46 707-711 (1987)). Phthalocyanine derivatives and other lipophilic dyes are known to bind to the membranes of cancer cells or enveloped viruses. When these compounds are activated with suitable wavelength of electromagnetic radiation, they produce singlet molecular oxygen (Kalyanaraman, *et al*., PNAS, 84 2999-3003 (1987)), which damages the membrane resulting in viral inactivation. With the addition of radiation sensitizers which comprise molecules which contain metal and/or halogen atoms, according to the present invention, use of chemical sensitizer molecules derived from membrane-binding molecules, such as described above in combination with suitable radiation produces free radicals in the proximity of the DNA/RNA/viral membranes. This results in destruction of the viral membranes or nucleic acid to inactivate the virus. Other organic dyes may be used as membrane-binding molecules, including porphyrins, especially haematoporphyrin and its derivatives, e.g. as described above.

The membrane-binding molecules may be fatty acid-based. Thus, halogenated or metal atom substituted fatty acids also may be utilized according to the present invention as radiation sensitizers. Fatty acids *per se* have been used in viral inactivation by Horowitz, *et al*. as disclosed in U.S. Patent No. 4,841,023. According to the present invention, these fatty acids may be utilized with sensitizer atoms to target viral membranes in plasma protein solutions, for example, and by subsequent activation with suitable radiation the free radicals are produced to inactivate the viral membrane.

Treatments of biological compositions with detergents are disclosed in U.S. Patent Nos. 4,820,805 and 4,764,369. The clinical application of psoralens in conjunction with photodynamic treatment is discussed by Adelson, *Scientific American* 50-57 (August 1988).

The following compounds are illustrative of sensitizers which contain or which may be modified to contain metal substituents or halogen substituents in accordance with the present invention:

**1. DNA or RNA Target Sensitizers**

**Psoralen Sensitizers:**

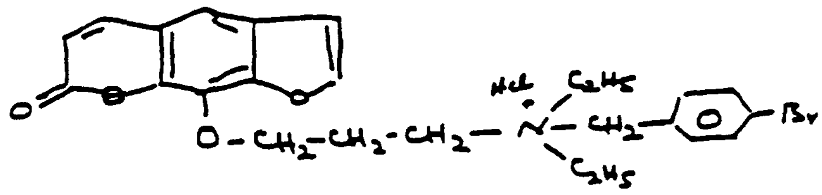

Compound # 1

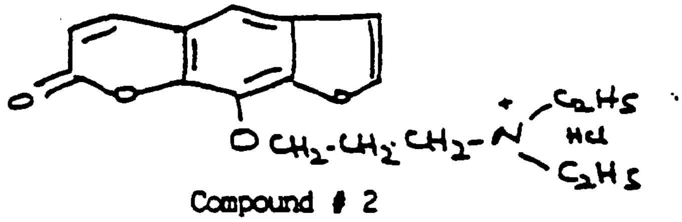

Compound # 2

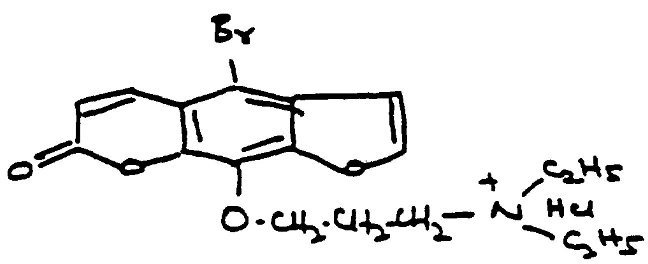

Compound # 3

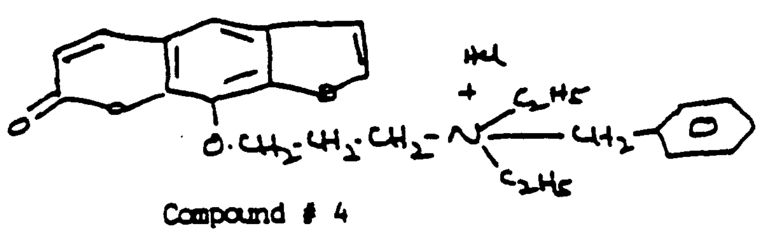

Compound # 4

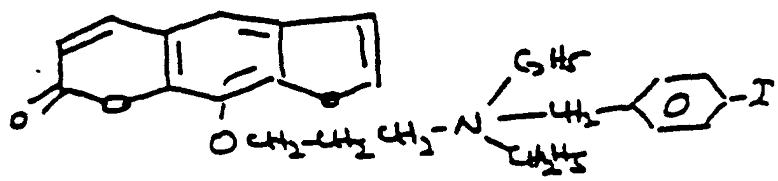

Compound # 5

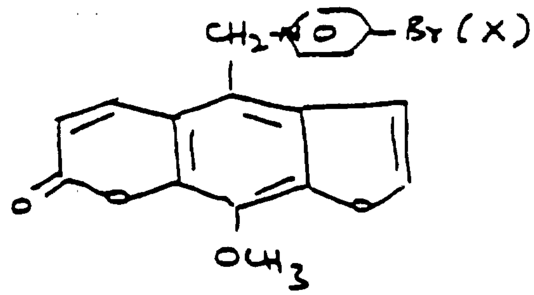

Compound # 6 X=I, Compound 6b

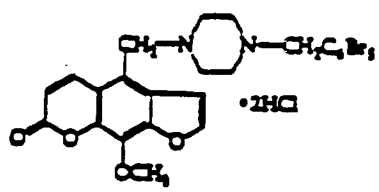

Compound # 7

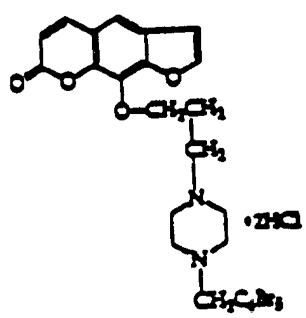

Compound # 8

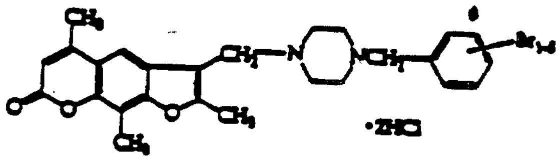

Compound # 9

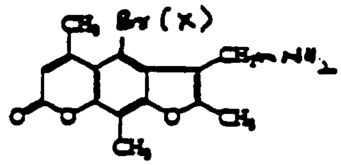

Compound # 10

X=I, Compound 10b

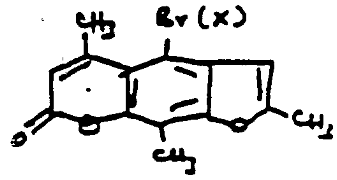

Compound 10c

X=I. Compound 10d

### 2. Membrane Target Sensitizers

**Phthalocyanine Sensitizers:**

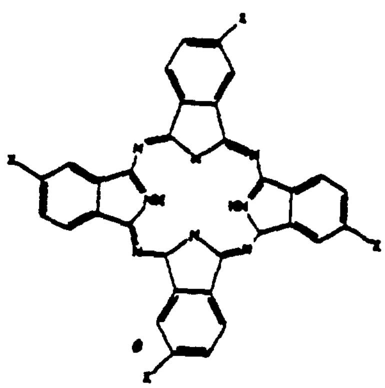

Compound # 11

Compound # 12

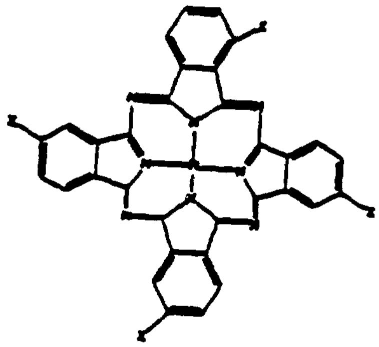

Compound # 13
Compound # 14
Compound # 15

**Merocyanine Sensitizers:**

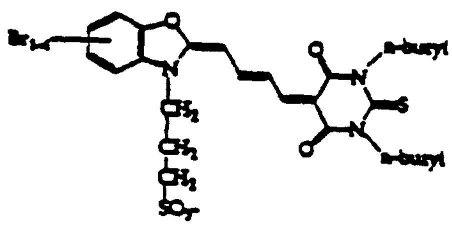

Compound 16

**Fatty acid Sensitizers:**

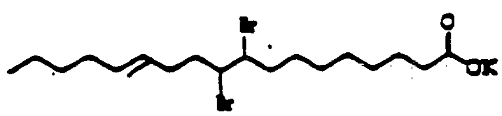

Compound # 17

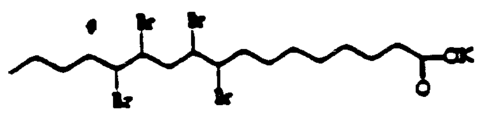

Compound # 18

Biological Dyes:

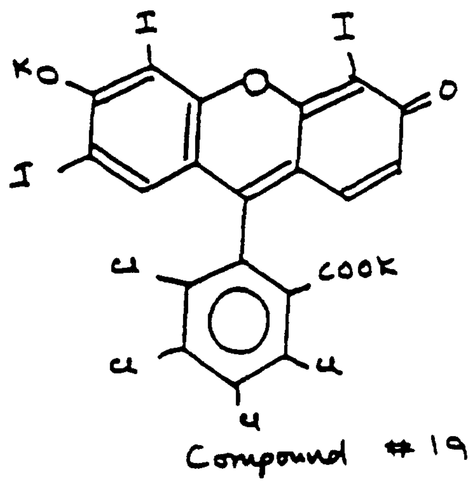

Compound #19

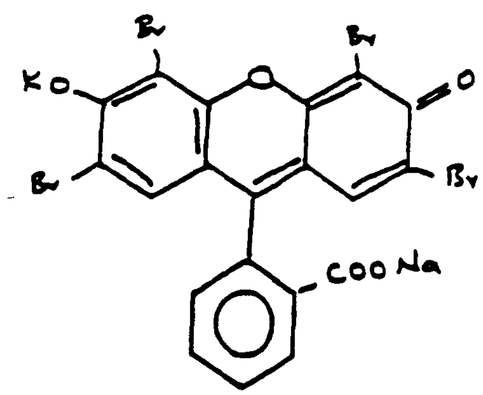

Compound #20

EXAMPLE 1

VIRUS INACTIVATION IN HYDRATED PLASMA

Stock solution of ϕ 6 phage was added to plasma to obtain the final virus concentrate of 1.2 x $10^7$ PFU/ml. Compound 20 (formula given above) was added to the mixture to give the sensitizer concentration of 0.5 mg/ml. After addition of the sensitizer, the solution was mixed on a mechanical shaker for 1 hour at room temperature. The sample was transferred to a plastic Petri dish (35x10mm) and irradiated in a Pantak HP 160 X-ray unit equipped with a Mo

target tube operating at 28 ma and 40 kv settings. Approximate radiation dose delivered was 353 kr. After irradiation the residual viral titre was measured by the plaque method. A phage containing irradiated sample was mixed with suitable phage host bacteria and 3 ml of melted soft agar. The mixture was poured over hard nutrient agar plate. After one day of incubation the lysed area stood out as plaque against the dense background. The plaques were counted with a colony counter. All samples were treated with X-ray radiation unless otherwise stated.

The initial titre of ϕ 6 virus 1.2 x $10^7$ PFU/ml in compound 20 (structure given above) and 0.5 mg/ml were used in hydrated plasma. The sample preparation and irradiation conditions were as described above.

For R-17 virus the starting viral titre of 4.2 x $10^8$ PFU/ml and compound No. 20 at 0.5 mg/ml were used. The results (see Figure) indicate that enhanced viral reduction is obtained with the use of sensitizers in combination with the X-ray radiation treatment.

EXAMPLE 2

VIRAL INACTIVATION IN HYDRATED AND LYOPHILIZED PLASMA IN PRESENCE AND ABSENCE OF ORGANIC SOLVENTS

The starting titre of ϕ 6 virus was 2.5 x $10^8$ PFU/ml in these samples. The plasma mixture was mixed with either 1% Tween® 80 detergent or 1% tri-(n-butyl)phosphate (TNBP) for 2 hours at room temperature. The samples were treated with 353 kr radiation dose in hydrated and lyophilized states as described above. The results indicate that the viral inactivation obtained with molybdenum X-ray radiation in hydrated and lyophilized plasma is substantially enhanced by addition of either organic solvent (TNBP) or detergent (Tween 80) individually or in combination. Other results relate to using a stock solution of R-17 virus added to plasma to give a starting titre of 6.2 x $10^8$ PFU/ml and 1% Tween 80 with 1% TNBP for two hours at room temperature. The hydrated and lyophilized samples were treated with 353 kr radiation dose and the final viral titre was determined as described above.

## Claims

**1.** A process for reducing viral, bacterial and/or parasitic contaminants in a composition comprising blood, a blood component, cell culture or a component of a cell culture, comprising the steps of:

mixing said composition in a liquid state with a chemical radiation sensitizer capable of targeting said viral, bacterial and/or parasitic contaminants;
treating the composition with an organic solvent or detergent; and
exposing the composition in the liquid or lyophilized state to electromagnetic radiation of a wavelength and intensity and for a period of time sufficient to activate said sensitizer, whereby the activation of the sensitizer reduces said contamination in said composition.

**2.** A process according to Claim 1, wherein the composition comprises virally contaminated blood plasma or blood plasma fraction and is treated with an organic solvent or detergent effective to decrease the viral titre of the blood plasma or fraction.

**3.** A process according to Claim 2, wherein said blood plasma faction comprises serum albumin, immune globulins, or a clotting factor.

**4.** A process according to Claim 3, wherein said blood plasma fraction comprises factor VIII.

**5.** A process according to Claim 1, wherein the cell culture comprises a recombinant plasma protein.

**6.** A process according to Claim 5, wherein the recombinant plasma protein is a recombinant serum albumin or recombinant clotting factor.

**7.** A process according to Claim 6, wherein the recombinant clotting factor is recombinant factor VIII.

**8.** A process according to any preceding Claim wherein said radiation comprises penetrating, ionizing radiation.

**9.** A process according to Claim 8, wherein said penetrating, ionizing radiation comprises X-rays or gamma-rays.

10. A process according to Claim 9, wherein said X-rays are produced by a metallic target source comprising molybdenum, palladium, rhodium, silver, tungsten, titanium, chromium, manganese, iron, cobalt, nickel, copper or zinc.

11. A process according to any preceding Claim wherein said chemical sensitizer comprises molecules which contain metal and/or halogen atom(s).

12. A process according to Claim 9 or 10, wherein said chemical sensitizer comprises molecules which contain metal and/or halogen atom(s) which increase the overall mass attenuation coefficient of the sensitizer to radiation from a predetermined X-ray target source.

13. A process according to Claim 11 or 12, wherein said metal atom(s) comprise platinum.

14. A process according to Claim 11, 12 or 13, wherein said chemical sensitizer molecules are derived from membrane-binding molecules.

15. A process according to Claim 14, wherein said membrane-binding molecules are fatty acid-based molecules or organic dyes.

16. A process according to Claim 15, wherein said membrane-binding molecules comprise porphyrins.

17. A process according to Claim 16, wherein said porphyrins comprise hematoporphyrin and hematoporphyrin derivatives.

18. A process according to any preceding Claim, wherein said organic solvent comprises tri-N-butyl phosphate.

19. A process according to any preceding Claim wherein said detergent comprises a non-ionic detergent.

20. A process according to any preceding Claim, wherein lyophilization is carried out to a residual moisture content of 10% or less.

**Patentansprüche**

1. Ein Verfahren zur Reduktion von viralen, bakteriellen und/oder parasitären Kontaminationen in einer Zusammensetzung, die. Blut, eine Blutkomponente, Zellkultur oder eine Komponente einer Zellkultur enthält, umfassend die Schritte von:

   Mischen besagter Zusammensetzung in einem flüssigen Zustand mit einem chemischen Strahlungssensibilisator, der dazu in der Lage ist, auf besagte Viren, bakterielle und/parasitäre Kontaminationen zu zielen;

   Behandeln der Zusammensetzung mit einem organischen Lösungsmittel oder Detergenz; und

   Aussetzen der Zusammensetzung im flüssigen oder lyophilisierten Zustand elektromagnetischer Strahlung einer Wellenlänge und Intensität und für einen Zeitraum der genügt, um besagten Sensiblisator zu aktivieren, wobei die Aktivierung des Sensibilisators die besagte Kontamination in besagter Zusammensetzung reduziert.

2. Ein Verfahren gemäß Anspruch 1, worin die Zusammensetzung viral kontaminiertes Blutplasma oder Blutplasmafraktionen umfaßt und mit einem organischen Lösungsmittel oder Detergenz behandelt wird, das wirksam ist, um den viralen Titer des Blutplasmas oder der Fraktion zu vermindern.

3. Ein Verfahren gemäß Anspruch 2, worin besagte Blutplasmafraktion Serum Albumin, Immunglobuline, oder einen Gerinnungsfaktor umfasst.

4. Ein Verfahren gemäß Anspruch 3, worin besagte Blutplasmafraktion Faktor VIII umfasst.

5. Ein Verfahren gemäß Anspruch 1, worin die Zellkultur ein rekombinantes Plasmaprotein umfasst.

6. Ein Verfahren gemäß Anspruch 5, worin das rekombinante Plasmaprotein ein rekombinantes Serum Albumin oder

rekombinanter Gerinnungsfaktor ist.

7. Ein Verfahren gemäß Anspruch 6, worin der rekombinante Gerinnungsfaktor ein rekombinanter Faktor VIII ist.

8. Ein Verfahren gemäß einem der vorhergehenden Ansprüche, worin besagte Bestrahlung durchdringende, ionisierende Strahlung umfasst.

9. Ein Verfahren gemäß Anspruch 8, worin besagte durchdringende, ionisierende Strahlung Röntgenstrahlen oder Gammastrahlen umfasst.

10. Ein Verfahren gemäß Anspruch 9, worin besagte Röntgenstrahlen durch eine metallische Strahlungsquelle produziert werden, die Molybdän, Paladium, Rhodium, Silber, Wolfram, Titan, Chrom, Mangan, Eisen, Kobalt, Nickel, Kupfer oder Zink umfasst.

11. Ein Verfahren gemäß einem der vorhergehenden Ansprüche, worin besagter chemischer Sensibilisator Moleküle umfasst, die Metall und/oder Halogenatome enthalten.

12. Ein Verfahren gemäß Anspruch 9 oder 10, worin besagter chemischer Sensibilisator Moleküle, welche Metall und/oder Halogenatom(e) enthalten, umfasst, welche den Gesamtmassenattenuationskoeffizienten des Sensibilisators für Bestrahlung aus einer vorbestimmten Röntgenstrahlen-Strahlungsquelle erhöht.

13. Ein Verfahren gemäß Anspruch 11 oder 12, worin besagte(s) Metallatom(e) Platin umfasst.

14. Ein Verfahren gemäß Anspruch 11, 12 oder 13, worin besagte chemische Sensibilisatormoleküle von membranbindenden Molekülen abgeleitet sind.

15. Ein Verfahren gemäß Anspruch 14, worin besagte membranbindende Moleküle auf Fettsäure basierende Moleküle oder organische Farbstoffe sind.

16. Ein Verfahren gemäß Anspruch 15, worin besagte membranbindende Moleküle Porphyrine umfassen.

17. Ein Verfahren gemäß Anspruch 16, worin besagte Porphyrine Hämatoporphyrin und Hämatoprophyrinderivate umfassen.

18. Ein Verfahren gemäß einem der vorhergehenden Ansprüche, worin besagtes organisches Lösungsmittel Phosphorsäure-tri-n-butylester umfasst.

19. Ein Verfahren gemäß einem der vorhergehenden Ansprüche, worin besagtes Detergenz ein nicht ionisches Detergenz umfasst.

20. Ein Verfahren gemäß einem der vorhergehenden Ansprüche, worin die Lyophilisierung bis zu einem Restfeuchtigkeitsgehalt von 10 % oder weniger ausgeführt wird.

## Revendications

1. Procédé pour réduire les contaminants viraux, bactériens et/ou parasitaires dans une composition contenant du sang, un composant du sang, une culture cellulaire ou un composant d'une culture cellulaire, comprenant les étapes consistant à:

   mélanger ladite composition à l'état liquide avec un sensibilisateur chimique de rayonnement capable de cibler lesdits contaminants viraux, bactériens et/ou parasitaires;
   traiter la composition avec un solvant organique ou un détergent; et
   exposer la composition à l'état liquide ou lyophilisé à un rayonnement électromagnétique ayant une longueur d'onde et une intensité suffisantes, et pendant une période de temps suffisante, pour activer ledit sensibilisateur, l'activation du sensibilisateur réduisant ainsi ladite contamination dans ladite composition.

2. Procédé selon la revendication 1, dans lequel la composition comprend un plasma sanguin ou une fraction de

plasma sanguin, contaminé par des virus, et est traitée avec un solvant organique ou un détergent efficace pour réduire le titre viral du plasma sanguin ou de la fraction.

**3.** Procédé selon la revendication 2, dans lequel ladite fraction de plasma sanguin comprend une albumine sérique, des immunoglobulines, ou un facteur de coagulation.

**4.** Procédé selon la revendication 3, dans lequel ladite fraction de plasma sanguin comprend le facteur VIII.

**5.** Procédé selon la revendication 1, dans lequel la culture cellulaire contient une protéine plasmatique recombinante.

**6.** Procédé selon la revendication 5, dans lequel la protéine plasmatique recombinante est une albumine sérique recombinante ou un facteur de coagulation recombinant.

**7.** Procédé selon la revendication 6, dans lequel le facteur de coagulation recombinant est le facteur VIII recombinant.

**8.** Procédé selon l'une quelconque des revendications précédentes, dans lequel ledit rayonnement comprend un rayonnement ionisant et pénétrant.

**9.** Procédé selon la revendication 8, dans lequel ledit rayonnement ionisant et pénétrant comprend des rayons X ou des rayons gamma.

**10.** Procédé selon la revendication 9, dans lequel lesdits rayons X sont produits par une source métallique cible comprenant du molybdène, du palladium, du rhodium, de l'argent, du tungstène, du titane, du chrome, du manganèse, du fer, du cobalt, du nickel, du cuivre ou du zinc.

**11.** Procédé selon l'une quelconque des revendications précédentes, dans lequel ledit sensibilisateur chimique comprend des molécules qui contiennent un ou plusieurs atome(s) de métal et/ou d'halogène.

**12.** Procédé selon la revendication 9 ou 10, dans lequel ledit sensibilisateur chimique comprend des molécules qui contiennent un ou plusieurs atome(s) de métal et/ou d'halogène qui augmentent le coefficient d'atténuation massique global du sensibilisateur vis-à-vis d'un rayonnement provenant d'une source cible de rayons X prédéterminée.

**13.** Procédé selon la revendication 11 ou 12, dans lequel ledit ou lesdits atome(s) de métal comprennent le platine.

**14.** Procédé selon la revendication 11, 12 ou 13, dans lequel lesdites molécules de sensibilisateur chimique sont dérivées de molécules de liaison membranaire.

**15.** Procédé selon la revendication 14, dans lequel lesdites molécules de liaison membranaire sont des molécules à base d'acide gras ou des colorants organiques.

**16.** Procédé selon la revendication 15, dans lequel lesdites molécules de liaison membranaire comprennent les porphyrines.

**17.** Procédé selon la revendication 16, dans lequel lesdites porphyrines comprennent l'hématoporphyrine et les dérivés d'hématoporphyrine.

**18.** Procédé selon l'une quelconque des revendications précédentes, dans lequel ledit solvant organique comprend le phosphate de tri-N-butyle.

**19.** Procédé selon l'une quelconque des revendications précédentes, dans lequel ledit détergent comprend un détergent non ionique.

**20.** Procédé selon l'une quelconque des revendications précédentes, dans lequel la lyophilisation est réalisée à une teneur en humidité résiduelle de 10% ou moins.

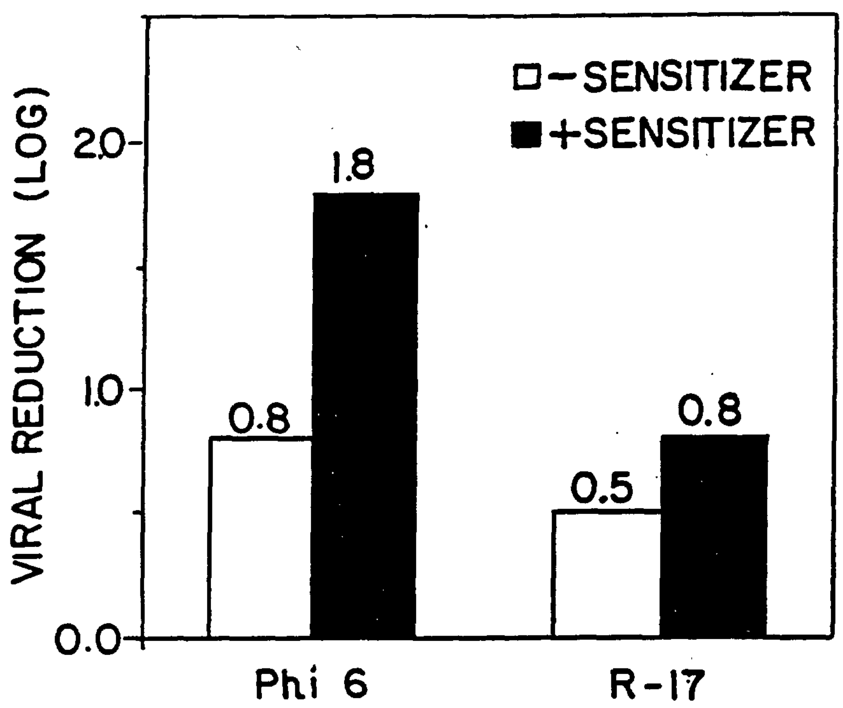
□-SENSITIZER
■+SENSITIZER
VIRAL REDUCTION (LOG)
2.0
1.0
0.0
1.8
0.8
0.8
0.5
Phi 6
R-17